# EUROPEAN PATENT SPECIFICATION

(11) **EP 1 217 999 B1**
(45) Date of publication and mention of the grant of the patent: **27.07.2005**
(21) Application number: 00969423.3
(22) Date of filing: 05.10.2000
(51) Int. Cl.: A61K 31/197, A61P 25/32

(54) **THE USE OF BACLOFEN IN THE TREATMENT OF ALCOHOLISM**
VERWENDUNG VON BACLOFEN ZUR BEHANDLUNG VON ALKOHOLISMUS
UTILISATION DU BACLOFENE POUR TRAITER L'ALCOHOLISM

(30) Priority: 08.10.1999 IT MI992107
(43) Date of publication of application: 03.07.2002
(73) Proprietor: Neuroscienze S.C.A.R.L., 09125 Cagliari (IT)
(72) Inventor: GESSA, Gian, Luigi, I-09125 Cagliari (IT); COLOMBO, Giancarlo, Centro CNR, I-09124 (IT); ADDOLORATO, Giovanni, Istituto di Medicina, I-00168 Roma (IT)
(74) Representative: Minoja, Fabrizio, Dr.
(86) International application number: PCT/EP2000/009750
(87) International publication number: WO 2001/026638

(56) References cited:
- BROADBENT J ET AL: "Differential effects of GABA(A) and GABA(B) agonists on sensitization to the locomotor stimulant effects of ethanol in DBA/2 J mice." PSYCHOPHARMACOLOGY, (1999 JAN) 141 (2) 197-205. , XP000965275
- HUMENIUK R E ET AL: "The role of GABAB receptors in mediating the stimulatory effects of ethanol in mice." PSYCHOPHARMACOLOGY, (1993) 111 (2) 219-24. , XP001036573
- BOISMARE F ET AL: "ARE GAMMA AMINOBUTYRIC-ACID-ERGIC RECEPTORS INVOLVED IN THE VOLUNTARY INTAKE OF ETHANOL BY THE RAT." ASSOCIATION FRANCAISE DES PHARMACOLOGISTES (FRENCH ASSOCIATION OF PHARMACOLOGISTS) FRANCO-ITALIAN MEETING OF PHARMACOLOGISTS, PARIS, FRANCE, JUNE 7-8, 1984. J PHARMACOL (PARIS). (1984 (RECD 1985)) 15 (4), 457-458. , XP001036594
- FILE S E ET AL: "BACLOFEN REVERSES THE SIGNS OF ETHANOL WITHDRAWAL IN RATS." MEETING OF THE BRITISH PHARMACOLOGICAL SOCIETY, SHEFFIELD, ENGLAND, UK, APRIL 18-20, 1990. BR J PHARMACOL. (1990) 100 (PROC SUPPL JUNE), 340P. , XP001036582
- DAOUST M ET AL: "GABA transmission, but not benzodiazepine receptor stimulation, modulates ethanol intake by rats." ALCOHOL, (1987 NOV-DEC) 4 (6) 469-72. , XP001036617
- KUZIEMKA-LESKA M ET AL: "Baclofen and AII 3-7 on learning and memory processes in rats chronically treated with ethanol." PHARMACOL., BIOCHEM. BEHAV., (1998). VOL. 62, NO. 1, PP. 39-43. CODEN: PBBHAU. ISSN. 0091-3057., XP001035246 Department of Pharmacology, Medical University in Bialystok, Bialystok
- FILE S E ET AL: "Effects of baclofen and nitrendipine on ethanol withdrawal responses in the rat." NEUROPHARMACOLOGY, (1991 FEB) 30 (2) 183-90. , XP001036625
- COLOMBO G ET AL: "Ability of baclofen in reducing alcohol intake and withdrawal severity: I--Preclinical evidence." ALCOHOLISM, CLINICAL AND EXPERIMENTAL RESEARCH, (2000 JAN) 24 (1) 58-66. , XP001036700
- ADDOLORATO G ET AL: "Ability of baclofen in reducing alcohol craving and intake: II--Preliminary clinical evidence." ALCOHOLISM, CLINICAL AND EXPERIMENTAL RESEARCH, (2000 JAN) 24 (1) 67-71. , XP001036708
- COLOMBO, G. (1) ET AL: "Ability of baclofen in reducing alcohol intake in alcohol -preferring sP rats and withdrawal severity in alcohol - dependent rats." ALCOHOLISM CLINICAL AND EXPERIMENTAL RESEARCH, (MAY, 2000) VOL. 24, NO. 5 SUPPLEMENT, PP. 37A. PRINT. MEETING INFO.: SCIENTIFIC MEETING OF THE RESEARCH SOCIETY ON ALCOHOLISM SANTA BARBARA, CALIFORNIA, USA JUNE 24-29, 2000 RESEARCH SOCIETY ON ALCOHOLI, XP001036738

## Description

The present invention relates to the use of Baclofen in the treatment of alcohol withdrawal syndrome and promotion of abstinence in alcoholics.

### TECHNOLOGICAL BACKGROUND

Alcoholism is a serious medical, social and economic problem facing almost all human societies worldwide. Alcoholism has been diagnosed in an estimated 5% of adult population in the Western countries, while a larger number of people have been classified as problem drinkers (i.e. people who drink alcohol at a level that is risky for their health). Medical interventions in the field of alcoholism are primarily aimed at a) relieving the consequences of alcohol withdrawal syndrome and b) arresting alcohol drinking, maintaining sobriety for as long as possible. Pharmacotherapy is conceived to provide a substantial contribution to these goals, facilitating the psychological support and social rehabilitation of alcoholic patients.

Several pharmacological substances acting on neurotransmitter systems affected by the action of alcohol have been studied, naltrexone (Volpicelli et al., 1992; O' Malley et al., 1992), acamprosate (Withworth et al, 1996), fluoxetine (Naranjo et al, 1994), may be mentioned, inter alia. Furthermore, gamma-hydroxybutyric acid (GHB) a compound with behavioral GABA-like effects (Colombo et al., 1998), proved able to decrease alcohol intake in rats (Agabio et al., 1998) and humans (Gallimberti et al., 1992) and to induce alcohol abstinence in alcoholics (Addolorato et al., 1996; 1998a; 1998b). In addition, GHB proved to be effective in treating alcohol withdrawal syndrome both in experimental animals (Gessa et al., 2000) and humans (Gallimberti et al., 1989), with a similar efficacy to diazepam (Addolorato et al., 1999b). It has been hypothesized that the effects of GHB on alcohol intake, craving and withdrawal syndrome are related to its alcohol-mimicking effect on the CNS (Colombo et al., 1995).

Baclofen, (beta-(4-chlorophenyl)-gamma-aminobutyric acid), a lipophilic derivative of GABA, is a potent and stereoselective GABA_{B} receptor agonist. At present it is used clinically to control spasticity (Davidoff, 1985).

Baclofen has already been tested in experimental animals to evaluate its capacity to induce a selective reduction of daily alcohol intake in Long Evans rats (Daoust et al., 1987); on the other hand, subsequent studies reported that a higher dose of baclofen stimulated daily alcohol intake during both acquisition (Smith et al., 1992) and maintenance (Smith et al., 1999) phases of alcohol drinking behaviour in Long Evans rats; finally, central administration of baclofen failed to alter alcohol intake in Wistar rats (Tomkins and Fletcher, 1996). With regard to alcohol withdrawal syndrome, File and colleagues (1991) reported that small doses of baclofen reduced the anxiety-like behaviours and tremors associated with alcohol withdrawal syndrome in alcohol-dependent rats; however, no effects on alcohol withdrawal tremors after baclofen administration were observed in mice (Humeniuk et al., 1994) and rhesus monkeys (Tarika and Winger, 1980).

Therefore, it was not possible to envisage a therapeutic effect of baclofen in the treatment of alcoholism on the basis of the aforementioned studies.

### DISCLOSURE OF THE INVENTION

Based on the clinical evidence available and on results obtained with reliable experimental models, it has been observed that baclofen may be successfully used in the treatment of alcoholism.

The results obtained by means of the present invention allow the overcoming of uncertainties as to inconsistencies and discrepancies in the above data which, when considered as a whole, made baclofen appear unsuitable for the specific aims.

Baclofen activity has been evidentiated in both a clinical study, as well as in Wistar rats previously rendered physically dependent on alcohol by the repeated administration of intoxicating doses of alcohol. Similar findings have been obtained using *Sardinian alcohol-preferring* (sP) rats, which are a predictive, reliable experimental model.

Accordingly, baclofen will be administered to alcoholic patients at daily dosages ranging from 10 to 50 mg, preferably from 15 to 30 mg, using conventional pharmaceutical compositions, preferably pharmaceutical compositions suited to oral administration.

The drug will be administered once or more times daily, and may be protracted for several weeks (for example, 3 to 6 weeks or more), in view of the fact that baclofen is well tolerated, is not toxic and does not induce addiction phenomena.

The invention will be now described in greater detail in the following Examples.

### Example 1: Effect of baclofen on alcohol withdrawal syndrome

### Animals

Male Wistar rats (Charles River, Calco, CO, Italy), weighing 275-300 g were used. After delivery, rats were left undisturbed for 7 days to acclimatize to new housing conditions. Animals were housed 5 per cage with wood chip bedding under an artificial light-dark cycle of 12/12 hr (light on at 7:00 hr), at a constant temperature of 22±2°C and relative humidity of 60%. Rats were given free access to water and standard laboratory food (MIL Morini, San Polo d'Enza, RE, Italy) throughout the entire experiment.

### Intoxication procedure

Rats were rendered physically dependent on alcohol by the method of Majchrowicz (1975). This consisted of 4 daily administrations of alcohol solution (20% w/v, in tap water) by intragastric gavage for 6 consecutive days, in order to maintain constant blood alcohol concentrations. Alcohol was administered at 6:00, 12:00, 18:00 and 24:00 hr. At the initial administration of the treatment, 4 g/kg alcohol were given to all rats. The assessment of subsequent doses was determined individually for each rat at the above administration times on the basis of the observed degree of intoxication using the intoxication-dose relationship conceived by Majchrowicz (1975). Six successive stages of intoxication were defined: neutrality, sedation, ataxia 1, 2 and 3, loss of righting reflex. Alcohol doses, ranging from 0 to 5 g/kg, were inversely related to the degree of intoxication. Assessment of the degree of intoxication and of the alcohol dose was decided by operators trained for the same evaluation criteria.

Rats were weighed once a day (at 9:00 hr). During chronic alcohol treatment, rats spent most of the time in a severe state of intoxication, unable to eat by themselves. Therefore, the loss in body weight was partially compensated by the daily oral administration (at 9:00 hr) of 20 g/kg liquid diet (Isomil, M&R, Zwolle, The Netherlands).

### Withdrawal assessment

Intensity of alcohol withdrawal signs was evaluated in each rat scoring a) spontaneous behaviour in its home cage for 10 sec, and then b) response to handling. Eleven separate items were scored using a 4-point scale (0 to 3, paralleling increased frequency of occurrence and degree of severity of items), modified from a scale described by Lal et al. (1988). The following items were rated: general activity, shakes, jerks, general tremors, head tremors, tail tremors, rigidity of muscle tone, tail rigidity, bracing posture, vocalization and spontaneous convulsions. The sum of the 11 values was the total score assigned to each rat on each observation. Scores of 8 to 9 indicated a neutrality state, corresponding to healthy and undrugged rats. Observation and scoring were carried out on a blind basis. Between observations, rats were left undisturbed in their home cage.

### Experimental design

Observations and scoring were carried out every hour for 11 consecutive hours starting at 15-hr after the last alcohol administration. Prior to beginning the observation and scoring, rats were randomly assigned to 4 groups of *n*=8 subjects each. Animals which convulsed prior to drug administration were excluded from the study. Baclofen [(±)-baclofen; Sigma Chemical Co., St. Louis, MO, USA] was dissolved in saline (added with a few drops of a 0.1N HCl solution) and injected ip at the doses of 0, 10, 20 and 40 mg/kg (injection volume: 10 ml/kg) immediately after the 15-hr observation period.

Two separate groups of rats received 0 (n=8) and 20 (n=9) mg/kg baclofen, dissolved and injected as described above, 16 hours after the last alcohol administration. One hour later, these rats were tested for susceptibility to audiogenic seizures, being placed in a cylindrical box of 60 cm diameter and exposed to 30-sec key shaking.

### Statistical analyses

Statistical evaluation of the daily amount of alcohol administered and the loss of rat body weight in each rat group was performed by one-way ANOVAs in the study testing the effect of baclofen on the intensity of alcohol withdrawal signs, and Mann-Whitney tests in the study testing the effect of baclofen on susceptibility to audiogenic seizures. Data concerning the effects of baclofen on the intensity of alcohol withdrawal signs were analyzed by a two-way (baclofen dose X time interval) ANOVA with repeated measures on time intervals, followed by the Newman-Keuls test in order to test group differences. Occurrence of audiogenic seizures was evaluated by means of Fisher's Exact test for a 2X2 Table [treatment (vehicle, baclofen) X seizure (presence, absence)].

### Results

Rats assigned to the various experimental groups did not differ in daily alcohol intake and loss of body weight during alcohol treatments. The average daily dose of alcohol administered was 9.7±0.3, 9.9±0.3, 9.9±0.4 and 10.1±0.4 g/kg [mean ± S.E.M.; F(3;188)=0.2858, P>0.05] in the rat groups receiving 0, 10, 20 and 40 mg/kg baclofen, respectively, in the study testing baclofen effect on the intensity of alcohol withdrawal signs, and 10.0±0.3 and 9.9±0.3 g/kg [mean ± S.E.M.; P>0.05 (Mann-Whitney test)] in the rat group receiving 0 and 20 mg/kg baclofen, respectively, in the study testing baclofen effect on susceptibility to audiogenic seizures. The average percentage of weight loss was 20.4±1.6, 19.0±0.7, 20.4±1.7 and 18.8±1.1 [mean ± S.E.M.; F(3;28)=0.4376, P>0.05] in the rat groups receiving 0, 10, 20 and 40 mg/kg baclofen, respectively, in the study testing baclofen effect on the intensity of alcohol withdrawal signs, and 20.0±1.2 and 19.5±1.3 [mean ± S.E.M.; P>0.05 (Mann-Whitney test)] in the rat group receiving 0 and 20 mg/kg baclofen, respectively, in the study testing baclofen effect on susceptibility to audiogenic seizures.

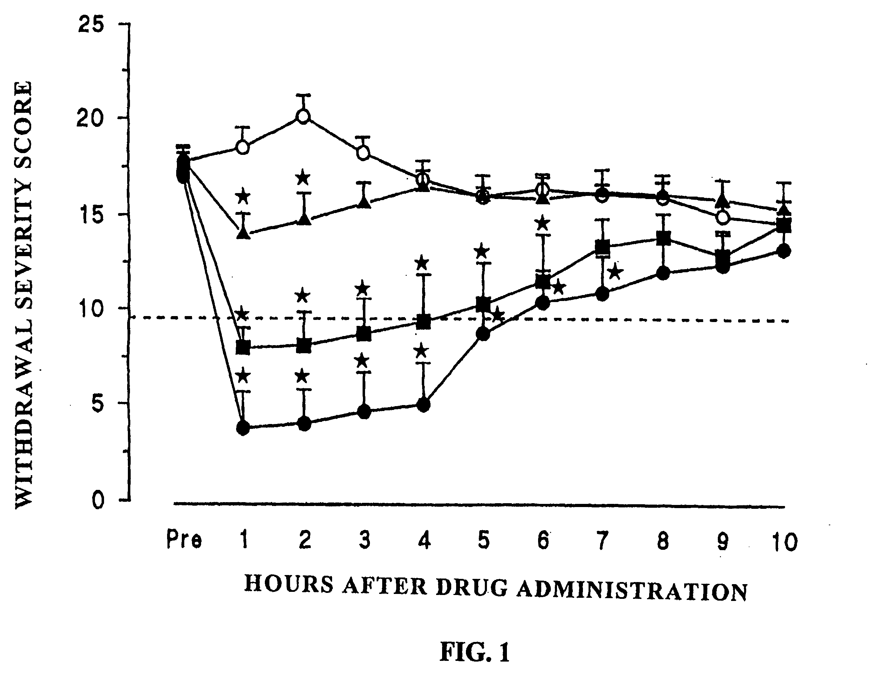

Baclofen administration resulted in a dose-dependent, significant reduction of the intensity of alcohol withdrawal signs in alcohol-dependent rats [F_{dose}(4;350)=8.04, P<0.0005] (Fig. 1). The *post-hoc* test indicated that reduction of alcohol withdrawal score lasted for 2, 6 and 7 hrs after drug administration in the rat groups treated with 10, 20 and 40 mg/kg baclofen, respectively. The highest dose tested (40 mg/kg) induced a marked degree of muscle relaxation and sedation, as shown by a withdrawal score lower than that obtained for healthy and undrugged rats. In contrast, at the dose of 20 mg/kg baclofen, no profound muscle flaccidity and loss of vigilance were observed, and the withdrawal score approached the neutrality-state set for 4-5 hours.

A dose of 20 mg/kg baclofen significantly (P<0.05, Fisher's Exact test) protected rats against audiogenic seizures. Indeed, 8 out of 8 rats in the vehicle-treated group and 5 out of 9 rats in the baclofen-treated group exhibited seizures after exposure to *key shaking.*

### Example 2: Effect of baclofen on voluntary alcohol intake

### Animals

Male sP rats, from the 42nd generation and approximately 6 months old, were used. Rat body weight ranged between 450 and 600 g. Rats were individually housed in standard plastic cages with wood chip bedding. The animal facility was under a reverse, artificial 12/12 hr light-dark cycle (light on at 21:00 hr), at a constant temperature of 22±2°C and relative humidity of 60%. Food pellets (MIL Morini, San Polo d'Enza, RE, Italy) were always available.

### Alcohol drinking procedure

Alcohol (10% v/v, in tap water) and tap water were offered under the two-bottle free choice regimen with unlimited access 24 hr/day. Bottles were refilled every day with fresh solution and their position interchanged at random to avoid development of position preference. Alcohol and water intakes were recorded daily, immediately before lights off. All rats used in the present study fulfilled the selection criteria adopted in this laboratory to qualify as sP rats (Colombo, 1997). Rats were habituated to handling, ip injection and frequent removal of bottles.

Rats were divided into 4 groups (n=7) matched for alcohol and water intakes over the 3 days preceding the start of drug treatment. Baclofen [(±)-baclofen; Sigma Chemical Co., St. Louis, MO, USA] was dissolved in 4 ml/kg saline and injected ip at the doses of 0, 2.5, 5 and 10 mg/kg once a day (20 to 30 min prior to light off) for 14 consecutive days. Alcohol, water and food intakes were monitored daily at 8:00-9:00 hr.

### Data Analyses

Data on baclofen effect on alcohol intake (expressed in g/kg), water intake (ml/kg), total fluid intake (ml/kg), food intake (g/kg) and preference ratio (percentage of alcohol solution consumed over total fluid intake) were analyzed by two-way (baclofen dose X days of treatment) ANOVAs with repeated measures on "treatment days". When appropriate, ANOVAs were followed by the Newman-Keuls test for post-hoc comparisons.

### Results

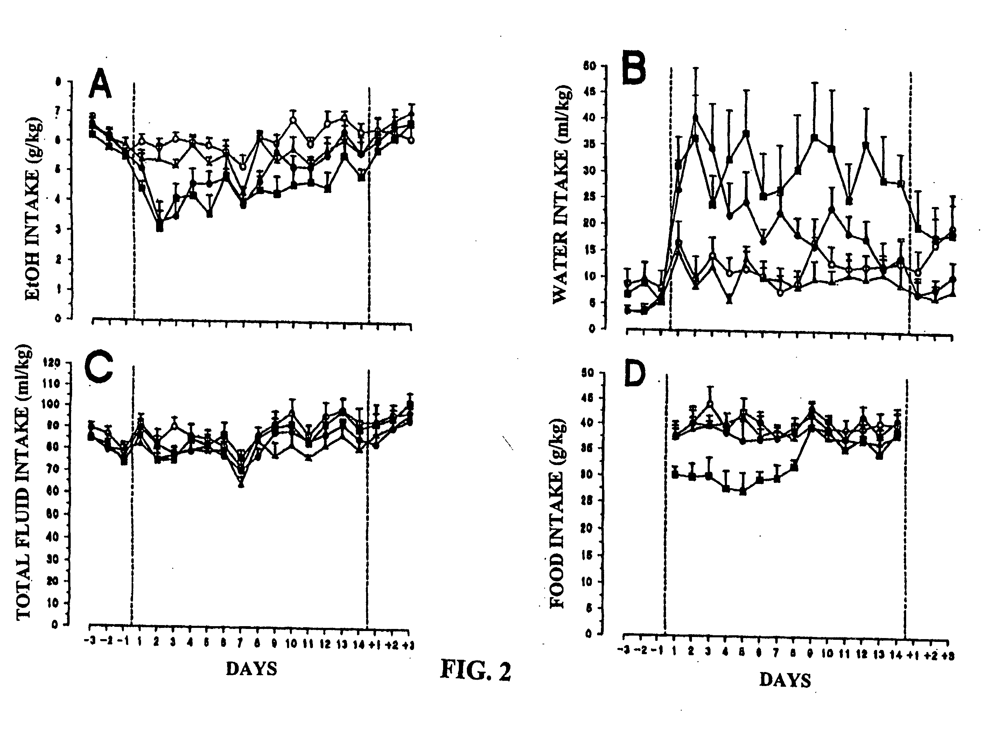

The repeated daily administration of baclofen induced a dose-dependent reduction of voluntary alcohol intake in sP rats [F_{dose}(3;312)=6.20, P<0.005] (Fig. 2, panel A). The magnitude of the reduction, compared to saline-treated rats and calculated over the entire treatment period, averaged approximately 10, 20 and 30% in the 2.5, 5 and 10 mg/kg baclofen-dosed rats. A dose-dependent, significant increase in water intake in baclofen-treated rat groups [F_{dose}(3;312)=5.12, P<0.01] (Fig. 2, panel B) compensated the reduction in alcohol consumption and left total fluid intake virtually unchanged [F_{dose}(3;312)=1.30, P>0.05] (Fig. 2, panel C). The preference ratio between alcohol solution and water consumed in baclofen-treated groups reflected the changes monitored in alcohol and water consumption [F_{dose}(3;312)=6.13, P<0.005] (data not shown). Finally, ANOVA revealed a significant effect of baclofen also on food intake [F_{dose}(3;312)=4.91, P<0.01]; however, as shown in Fig. 2, panel D, the reducing effect of baclofen was limited to the highest dose tested and to the first half of the treatment period. When baclofen administration was discontinued, both alcohol and water intakes returned immediately to control levels (Fig. 2).

### Example 3: clinical tests

A total of 10 male patients of mean age: 44.0±10.1 yrs with current alcoholism according to DSM IV criteria by American Psychiatric Association (1944) were studied. Baclofen was orally administered for 4 weeks, at the dose of 15 mg/day refracted in 3 times/day for the first 3 days, increasing the dose to 30 mg/day refracted in 3 times/day for the remaining 27 days.

The effects of the treatment were evaluated by the Alcohol Craving Scale (ACS) at the start of the study (T0) and subsequently on a weekly basis until completion of treatment. (T1 - T4). ACS is a questionnaire containing 11 items, each of which requires a yes or no answer, corresponding to 1 or 0 points, respectively, and 3 multiple choice questions, to which a score of 1 is attributed for affirmative answers; the maximum craving score was therefore 14 (Gallimberti et al, 1992; Addolorato et al, 1998b). Moreover abstinence from alcohol was evaluated on the basis of the patient's self-evaluation and of family member interview, and determination of blood alcohol concentration and of alcohol in saliva by QED (Enzymatics Inc., Horsham, UK) at each outpatient control, and on the basis of the main biological markers of alcohol abuse (aspartate aminotransferase-AST-, alanine aminotransferase-ALT-, gamma glutamyltranspeptidase-GGT-, mean cellular volume-MCV-) performed at the start and at the end of the study. Finally a self-reported alcohol intake was recorded as the mean number of standard drinks consumed per day (one standard drink = 12 grams of absolute alcohol) (Secretary of Health and Human Services, 1997).

### Results

Of the ten individuals recruited, one dropped out and was therefore excluded from the statistical analysis. Of the nine who completed the study, two continued to drink alcohol, although they substantially reduced their daily drinks from the first week of treatment (namely, the median value of their daily drinks was reduced from 8, as recorded prior to the start of the treatment, to 2, and then remained stable throughout the experimental period. Remarkably, the other seven subjects achieved and maintained a complete abstinence throughout the experimental period.

Baclofen was revealed as being effective in reducing alcohol craving from the first week of drug administration (ACS median score and [range]: T0: 9 [3-14] vs. T1: 3 [0-8]; p<0.01); subsequently the ACS median value was stable at the different times of observation (table).

No notable difference in ACS median score was found between the abstinent patients and subjects who continued to drink at any time evaluated (table).

The most common sensation reported by patients was the disappearance of obsessive thinking about alcohol after a few days of baclofen administration. Obsessive thinking refers to a mental state in which alcoholic patients, especially in the first stage of treatment, have a constant internal dialogue about whether to use alcohol or to resist. One of these patients had experienced GHB anti-craving effects several years previously, but reported no change in his obsessive thoughts about alcohol with GHB therapy.

Comparison of laboratory data obtained both prior to and following baclofen administration, a significant decrease in values of GGT (T0: 71.7±44.2 U/l vs T4: 31.2±18.0 U/l, p<0.01), of AST (T0: 54.7±13.4 U/l vs T4: 23.5±10.0 U/l, p<0.01), ALT (T0: 55.1±17.4 U/l vs T4: 21.7±10.2 U/l, p<0.01) and MCV (T0: 96.3±3.4 µµ² vs T4: 93.6±2.4 µµ², p<0.01) was found.

As far as side-effects are concerned, no serious systemic or single-organ events leading to drug cessation were reported and no patients discontinued the drug. In one patient the daily dose of the drug was reduced to 15 mg per day from the 2^{nd} week of treatment due to headache, difficulty in concentrating, lack of appetite and sedation. Tolerability was fair in all patients. No patients referred euphoria or other pleasant effects caused by the drug. No subjects showed craving for the drug; at drug discontinuation, no drug withdrawal syndrome or side effects due to drug suspension was observed.

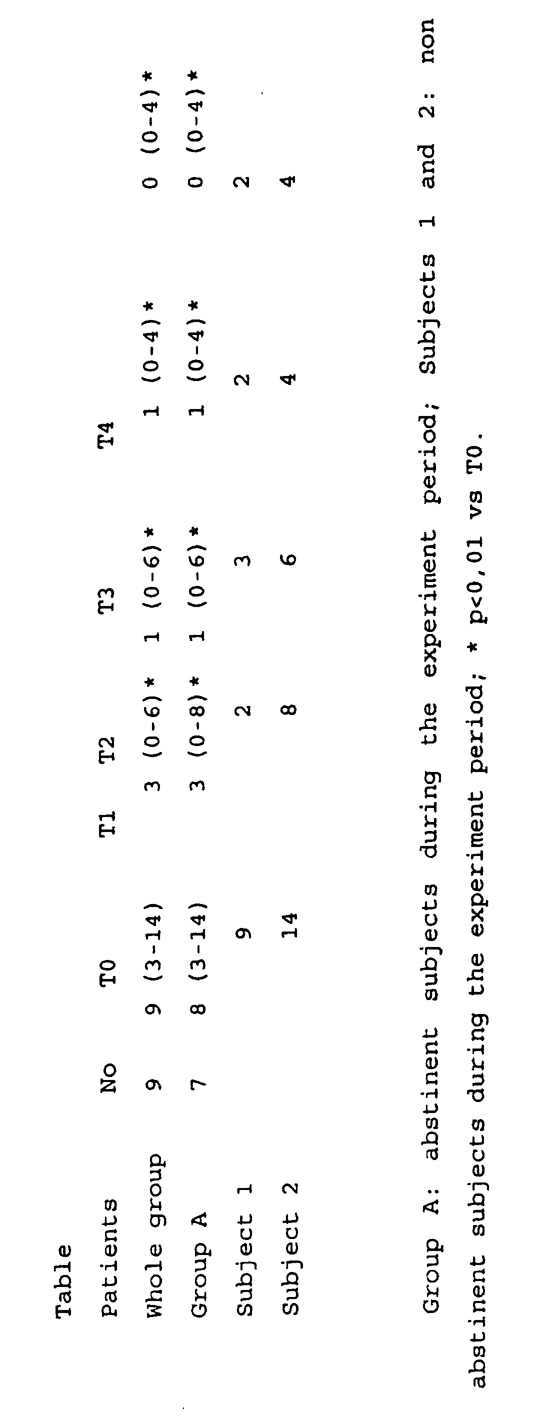

### REFERENCES

Anton RF (1996), Alcohol Alcohol 31 (suppl 1): 43-53; 1996.
Erickson CK (1996), Alcohol Alcohol. 31 (suppl 1): 5-11.
Secretary of Health and Human Services (1997), NIH publication no 97-4017.
Volpicelli JR, et al. (1992), Arch Gen Psychiatry 49:876-880.
Naranjo CA, et al. (1994), Int Clin Psychopharmacol 9:163-172.
Colombo G, et al. (1998), Physiol Behav 64:293-302.
Agabio R, et al. (1998), Alcohol Alcohol 33:465-474.
Gallimberti L, et al. (1992), Alcohol Clin Exp Res 16:673-676.
Addolorato G, et al. (1996), Alcohol Alcohol 31:341-345.
Addolorato G, et al. (1998a), Lancet 351:38; 1998.
Addolorato G, et al. (1998b), Drug Alcohol Depend 53:7-10.
Gessa GL, et al. (2000), Alcohol 20:271-276, 2000.
Gallimberti L, et al. (1989), Lancet ii:787-789.
Addolorato G, et al. (1999b), Alcohol Clin Exp Res (in fase di stampa).
Colombo G, et al. (1995), Physiol Behav 57:105-111.
Davidoff RA (1985), Ann Neurol 17:107-116.
Daust M, et al. (1987), Alcohol 4:469-472.
Smith BR, et al. (1992), Alcohol Alcohol 27:227-231
Smith BR, et al. (1999), Alcohol 17:231-240.
Tomkins DM, Fletcher PJ (1996), Behav Pharmacol 7:85-93.
File SE, et al. (1991), Neuropharmacology 30:183-190.
Humeniuk RE, et al. (1994), Pharmacol Biochem Behav 49:561-566.
Tarika JS, Winger G (1980), Psychopharmacology 70:201-208.
Lal H, et al. (1988), J Pharmacol Exp Ther 247:508-518.
Colombo G (1997), Alcohol Alcohol 32:443-453.
Gallimberti L, et al. (1992), Alcohol Clin Exp Res 16:673-676.

## Claims

1. The use of baclofen for the preparation of a medicament for the treatment of alcoholism in humans.

## Patentansprüche

1. Verwendung von Baclofen zur Herstellung eines Arzneimittels zur Behandlung von Alkoholismus in Menschen.

## Revendications

1. Utilisation du baclofene pour la préparation d'un médicament pour traiter l'alcoolisme chez l'homme.
